(19) Europäisches Patentamt
European Patent Office
Office européen des brevets

(11) **EP 3 295 927 A1**

(12) **EUROPEAN PATENT APPLICATION**

(43) Date of publication:
**21.03.2018 Bulletin 2018/12**

(21) Application number: **16382437.8**

(22) Date of filing: **19.09.2016**

(51) Int Cl.:
*A61K 8/73* *(2006.01)*          *A61K 35/74* *(2015.01)*
*A61Q 19/08* *(2006.01)*          *A61K 8/99* *(2017.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
• **Institut Univ. de Ciència i Tecnologia, S.A.
  08100 Mollet del Vallès Barcelona (ES)**
• **Bionos Biotech, S.L.
  46026 Valencia (ES)**

(72) Inventors:
• **DERONCELÉ THOMAS, Víctor Manuel
  08100 MOLLET DEL VALLÈS (ES)**

• **MONTILLA AREVALO, Rafael
  08100 MOLLET DEL VALLÈS (ES)**
• **CASTELLS BOLIART, Josep
  08100 MOLLET DEL VALLES (ES)**
• **SÁNCHEZ SÁNCHEZ, Ana Virginia
  46026 VALENCIA (ES)**
• **GONZÁLEZ FERNÁNDEZ, David
  46026 VALENCIA (ES)**
• **MULLOR, José Luis
  46026 VALENCIA (ES)**

(74) Representative: **Oficina Ponti, SLP
  C. de Consell de Cent 322
  08007 Barcelona (ES)**

(54) **USES OF AN EXOPOLYSACCHARIDE-PROTEIN COMPLEX OBTAINED FROM A BACTERIUM**

(57)   The present invention relates to the use of at least one exopolysaccharide-protein complexobtained from a bacterium, preferably said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.,* comprising: (i) a crude exopolysaccharide, and (ii) exopolysaccharide-associated proteins for the cosmetic treatment of the skin and/or the hair or as an antioxidant agent.

EP 3 295 927 A1

**Description**

FIELD OF THE INVENTION

The present invention relates to an exopolysaccharide-protein complex obtained from a bacterium. In particular, the present invention relates to the uses of said exopolysaccharide-protein complex obtained from a bacterium. More particularly, the present invention relates to the cosmetic use on the skin and/or hair and the use as an antioxidant agent of said exopolysaccharide-protein complex obtained from a said bacterium.

BACKGROUND OF THE INVENTION

An increasing number of bacteria have been isolated from hypersaline environments throughout the past 20 years and have found to be an extraordinary source of innovative molecules with unusual biological properties. For instance, sources of bioactive compounds and compatible solutes with biological activities ranging from antioxidant, sunscreen, and antibiotics actions. These properties have been developed as adaptative mechanisms to respond to the salt-stress and other extreme conditions (desiccation, radiation, oligotrophy, etc.).

Among these, bacteria belonging to the genus *Halomonas,* have been identified as able to produce large amounts of biologically active exopolysaccharides (EPS) as a strategy for growth, adhering to solid surfaces, and to survive under adverse conditions. A close investigation of some of these biopolymers produced by *Halomonas* species revealed that they could be of powerful value in foods, feeds, cosmetics, pharmaceutical and chemical industries (Poli, Anzelmo, & Nicolaus, 2010). For instance, Levan has been credited as one of the most promising polysaccharides for several industrial applications (Donot, Fontana, Baccou, & Schorr-Galindo, 2012) and, potential uses of Levan produced by *H. simyrnensis* AAD6T, as a bioactive polymer, were reported (Costa et al., 2013)

Microbial EPS are high molecular weight biopolymers, presenting an extreme diversity in terms of chemical structure and composition. Polysaccharides are the most abundant component of the EPS but previous electron microscopy studies (Nevot, Deroncele, Lopez-Iglesias, et al., 2006; Nevot, Deroncele, Messner, Guinea, & Mercade, 2006), heavily emphasized that other macromolecules such as proteins can be found too.

Along with the general properties like bio-compatibility, bio-degradability, renewability, flexibility, and eco friendliness, EPS also could be of powerful value in the cosmetic industry by providing a new way to protect the skin from environmental injuries. One special extracellular exopolysaccharide (Deepsane) has been isolated from cultured *Alteromonas macleodii* (Cambon-Bonavita, Raguenes, Jean, Vincent, & Guezennec, 2002). Deepsane is now produced in amounts suitable for use in the personal care and clinical studies have shown that Deepsane significantly reduces ICAM-1 expression in keratinocytes while also protecting Langerhans cells, which are essential players in the skin's immune system.

The number of reports on the evaluation of the effects of EPS addition in cosmetics searching for new cosmetic formulation is very limited, most of them devoted to algae or microalgae. New microbial exopolysaccharides could conquer the traditional polysaccharides market in the case of using cheap substrates; development of low costly downstream processes; better functional or novel properties. According to the report of Food and Drug Administration and Personal Care Products Council, 19 microbial polysaccharides are currently used in cosmetic formulations (Radchenkova et al., 2015). Numerous studies have shown that exopolysaccharides isolated from microorganisms could affect the immune responses both in vivo and in vitro and have the potential of being immunomodulators (Gorska et al., 2014; Shao et al., 2014). Biological activities, such as antiviral, antitumor, immunostimulating, anti-inflammatory, anticoagulant, anti-mutagenic, anticancer and antioxidant have been now evaluated in the cosmetic field; and novel and safe exopolysaccharides has become a popular research topic tor skin cosmetic applications. Recent scientific evidence has served to validate the efficacy of EPSs for supporting beauty from inside, in the form of nutricosmetics, and from the outside, as cosmeceuticals.

Exposure of skin to ultraviolet light has been shown to have a number of deleterious effects including photoaging, photoinduced DNA damage and photo-immunosuppression, which can lead to the development of skin cancer (Maini, Fahlman, & Krol, 2015). Cells exposed to UVB radiation show excessive levels of reactive oxygen species (ROS), DNA damage, activation of death receptors, and secretion of inflammatory cytokines, which all contribute to UVB-induced apoptosis. ROS-mediated oxidative damage causes DNA modification, lipid peroxidation, and secretion of inflammation cytokines. In DNA, 2'-deoxyguanosine is easily oxidized by ROS to 8-hydroxy-2'-deoxyguanosine (8-oxo-dG). 8-oxo-dG is a substrate for several DNA based excision repair systems and is released from cells after DNA repair. This modified nucleotide is used extensively as a biomarker of oxidative damage in DNA (Pauloin, Dutot, Joly, Warnet, & Rat, 2009).

These deleterious effects could be prevented with the use of UV protective functional ingredients. More and more skin care products are incorporating these ingredients into their formulas. Natural products are attracting much interest for a range of health benefits, including protection against photoinduced damage to skin. Some, such as green tea polyphenols, have strong UV absorption characteristics and act as sunscreens (Mishra, Mishra, & Chattopadhyay, 2011). Furthermore, as UVA radiation creates oxidation-induced damage to skin, dietary antioxidants such as ascorbic acid and carotenoids have been studied for their protective effects.

Evolution has provided the human organism with protection mechanisms against the harmful effects of free radicals, based on a complex defense system formed by antioxidant agents. These can be enzymatic mechanisms termed endogenous antioxidant agents (including enzymes SOD, catalase, glutathione peroxidase, coenzyme Q) or exogenous antioxidant agents which are responsible for making them available to our cells. Thus, it is important that the industry provides appropriate antioxidant delivery cosmetic or food products that can fulfill the need for exogenous antioxidant agents.

On the other hand, exposure of skin to UV light has been shown to have a number of deleterious effects. While most UV-C is absorbed by the ozone layer and does not reach the earth, both UV-A and UV-B radiations are considered as carcinogens (cancer causing agents), though their methods of action are distinct (Woelfle et al., 2011). Both types of UV light (A and B) are able to penetrate into different depths of the skin and hence affect different cells in the epidermis and dermis: UV-B radiation is mainly absorbed by epidermal components such as proteins or deoxyribonucleic acid (DNA), whereas UV-A radiation penetrates deeply into the skin and reaches the lower epidermis and dermal fibroblasts (Oresajo, Krutmann, Yatskayer, & Grether-Beck, 2012). The extent of their effect also varies, with UV-B being described as the most carcinogenic among all types of solar radiation. UV-B radiation's main deleterious effect is DNA damage caused by its direct interaction with the DNA molecule, while UV-A radiation's toxicity mainly comes from oxidative damage to skin cell components.

Like skin, the hair is exposed every day to a range of harmful effects such as sunlight, pollution, cosmetic treatments, grooming practices and cleansing. The UV components of sunlight damage human hair, causing fiber degradation. UV-B attacks the melanin pigments and the protein fractions (keratin) of hair and UV-A produces free radical/reactive oxygen species (ROS) through the interaction of endogenous photosensitizers. Antioxidant formulations for hair cosmetics that facilitate repair and prevent adverse effects on the capillary structure include antioxidants in their compositions to reduce the adverse effects on hair fiber. The antioxidants can also interrupt radical-chain processes, protect against oxidative damage and help to repair skin/hair systems.

Various exopolysaccharides described in the prior art have been used for cosmetic purposes. Applications WO2012072245A2 (Courtois, Thollas, Delgado, Cebrian, & Soley, 2012) and WO2016066857A1 (Mercadé Gil Mª, Carrion Fonseca, & Montes López Mª, 2015) disclose the use of an exopolysaccharide secreted by bacterial strains of the genus *Pseudoalteromonas* in cosmetic or dermopharmaceutical compositions for the treatment and/or care of the skin, mucous membranes, hair and/or nails. Application WO2015117985A1 (Mercadé Gil Mª et al., 2015) discloses an EPS derived from *Pseudomonas* sp., useful as cryoprotectant, emulsifier, thickening, stabilizing dermoprotective agent; and for increasing skin elasticity. Application WO2015063240A1 (García Sanz, Ferrer Montiel Antonio, Soley Astals, & Alminana Domenech, 2015) describes the cosmetic and/or dermopharmaceutical use of an EPS produced by *Halomonas anticariensis,* specifically, for the treatment and/or care of the skin, and in particular, its use for inflammation, lipolysis, lipid accumulation and skin firmness. However, it is important to note that these patent documents only disclose the use of the purified polysaccharidic moiety of the extracellular material produced for these bacteria and do not encompass the use of the combination of extracellular polysaccharides and selected associated-protein (exopolysaccharide-proteins complex).

WO 2009/127057 A1 (Loing, Briatte, Vayssier, Beaulieu, & Dionne, 2009) disclose a skin care composition comprising one exopolysaccharide derived from a microbial mat. The patent discusses the use of different compositions for cosmetic or therapeutic approaches and provided examples for evaluate the effects of the EPSs on the synthesis of hyaluronic acid, lipid synthesis, among others related with cosmeceuticals applications. However, this patent does not disclose the use of EPS with selected associated proteins.

Thus, the above prior art known processes relating to the production of EPS from microbial sources do not disclose the combined effect of polysaccharide fraction with selected extracellular EPS-associated proteins.

The present inventors have surprisingly found that the non-toxic and biodegradable exopolysaccharide-protein complex secreted by a bacterium, preferably said bacterium being from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.,* counteracts the malignant effects of UV irradiation and has a powerful antioxidant activity, scavenging and detoxifying the cell from free radicals through the metabolic pathways of glutathione and 8-hydroxy-2'deoxyguanosine. Also, inventors have found that the composition is non-irritating for the skin and has a very good cutaneous compatibility and at the same time increases endogenous keratin synthesis. For this reason, this exopolysaccharide-protein complex can be proposed for use as functional ingredient in skin and/or hair care formulations to prevent and modulate oxidative skin damages and as hair restorer ingredient.

BRIEF DESCRIPTION OF THE DRAWINGS

Fig. 1 shows sodium dodecylsulfate polyacrylamide gel electrophoresis (SDS-PAGE) gel of EPCx. Analysis from SDS-PAGE gel indicates that the two principal bands have molecular weights of -33 kDa and -55 kDa, respectively.

Fig. 2 shows the percentage of cells with UVB induced-thymine dimers, normalized to the irradiated control -without treatment. *Represents statistical significance with p-value <0.05.

Fig. 3 shows the histograms of one replicate of control non-irradiated cells (A) and control irradiated cells (B). X axis shows the intensity of fluorescence, and Y axis shows the number of cells. Windows discriminate between cells without thymine dimers (negative window) or with thymine dimers (positive window).

Fig. 4 shows a graphical representation of the number of cells with thymine dimers, after treating cells with EPCx at 1 $\mu$g/ml and 10 $\mu$g/ml and irradiating with UV-B light.

Fig. 5 shows the antioxidant activity of EPCx. A: Concentration of glutathione disulphide (GSSG), after treating cells with EPCx at 10 $\mu$g/ml. B: GSH/GSSG ratio, after treating cells with EPCx at 10 $\mu$g/ml. C: Concentration of 8OHdG, after treating cells with EPCx at 1 $\mu$g/ml and 10 $\mu$g/ml.

Fig. 6 shows KRT1 concentrations in control samples and treated with 1 $\mu$g/ml and 10 $\mu$g/ml concentrations samples. The results are statistically significant with a p-value <0.05.

SUMMARY OF THE INVENTION

A first object of the invention relates to the use of at least one exopolysaccharide-protein complex obtained from a bacterium, preferably said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.,* comprising: (i) a crude exopolysaccharide, and (ii) exopolysaccharide-associated proteins for the cosmetic treatment of the skin and/or the hair.

A second object of the invention relates to the use of at least one exopolysaccharide-protein complex obtained from a bacterium, preferably said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.,* comprising: (i) a crude exopolysaccharide, and (ii) exopolysaccharide-associated proteins, as an antioxidant agent.

DETAILED DESCRIPTION OF THE INVENTION

DEFINITIONS

In order to facilitate the comprehension of this invention, the meanings of some terms and expressions as used in the context of the invention are included.

As used herein, the term "isolated" should be considered to mean material removed from its original environment in which it naturally occurs, for example, in this instance a bacterial strain from hypersaline environment.

As used herein, the term "EPS" or "exopolysaccharide" or "EPCx" should be understood to mean high molecular weight polymers that are composed of sugar residues and expressed by bacteria.

As used herein, the term "hypersaline" refers to a kind of extreme environments that have salt concentrations much greater than that of seawater, often close to or exceeding salt saturation.

As used herein, the term "cosmeceutical composition" refers to a composition that is employed as both a cosmetic composition and as a pharmaceutical composition.

As used herein, the term "an antioxidant component," or "antioxidant," means a substance that can delay the onset or slow the rate of oxidation of oxidizable materials. Antioxidant component and antioxidant are used herein interchangeably. An antioxidant is generally a substance that participates in chemical, physiological, biochemical, or cellular processes that inactivate free radicals and/or active-oxygen species (singlet oxygen, hydrogen peroxide, hydroxyl radical, etc.), or prevent free radical-initiated chemical reactions.

EMBODIMENTS

In a first aspect, the present invention relates to the use of at least one exopolysaccharide-protein complex obtained from a bacterium, wherein said exopolysaccharide-protein complex comprises: (i) a crude exopolysaccharide, and (ii) exopolysaccharide-associated proteins for the cosmetic treatment of the skin and/or hair, preferably wherein said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.*

In the context of the present invention, the term "comprising" or "comprise(s)" should be understood as the components of the exopolysaccharide-protein complex are not limited to (i) a crude exopolysaccharide, and (ii) exopolysaccharide-associated proteins.

In another embodiment, the present invention relates to the use of at least one exopolysaccharide-protein complex obtained from a bacterium, wherein said exopolysaccharide-protein complex consisting of: (i) a crude exopolysaccharide, and (ii) exopolysaccharide-associated proteins for the cosmetic treatment of the skin and/or hair, preferably wherein said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.*

In the context of the present invention, the term "consists of" or "consisting of" should be understood as the components of the exopolysaccharide-protein complex are exclusively (i) a crude exopolysaccharide, and (ii) exopolysaccharide-associated proteins.

In a preferred embodiment, the crude exopolysaccharide contained in said exopolysaccharide-protein complex obtained from a bacterium, is a heteropolymer comprising or consisting of:

-    glucose units,
-    galactose units,
-    uronic acid units, and
-    other sugar units selected from rhamnose, glucosamine and a mixture of rhamnose and glucosamine.

In a further preferred embodiment, the crude exopolysaccharide contained in said exopolysaccharide-protein complex obtained from a bacterium, comprises or consists of: (a) 30 to 60 wt% glucose, (b) 30 to 50 wt% galactose, (c) 5 to 10 wt % uronic acids and (d) 1 to 10 wt% of other sugar units, providing that the sum of the components in the crude exopolysaccharide is 100 wt%, i.e. the components (a), (b), (c) and (d) must amount 100 wt% if components (a), (b), (c) and (d) are the only components in the exopolysaccharide-protein complex or the components (a), (b), (c) and (d) plus any further component(s) must amount 100 wt% if components (a), (b), (c) and (d) are not the only components in the exopolysaccharide-protein complex.

In a further preferred embodiment, the crude exopolysaccharide contained in said exopolysaccharide-protein complex obtained from a bacterium, further comprises sulphate at a concentration from 2 to 10 wt%.

In a further preferred embodiment, in the exopolysaccharide-protein complex, the exopolysaccharide-associated proteins include two bands of molecular weight as obtained by SDS-PAGE. In particular, the EPCx has a protein profile comprising at least, according to the SDS-PAGE technique, 12 detectable bands, including two principal bands corresponding, respectively, to molecular weights (approximate molecular weights given in relation to molecular standards, notably provided by Bio-Rad Laboratories) ranging between:

-    band 1: 30 kDa and 40 kDa, in particular 33kDa;
-    band 2: 51 kDa and 60 kDa, in particular 55kDa;

In a further preferred embodiment, said use of the exopolysaccharide-protein complex as previously defined is in the cosmetic field, in particular for the cosmetic treatment of the skin, more particularly, for protecting the skin from ultraviolet radiation due to sun exposure.

The determination of the protective effects exopolysaccharide-protein complex preparations is shown in the example section. Briefly, EPCx complexes were tested at concentrations between 1 to 10 $\mu$g/ml, in order to evaluate the protective effect on human keratinocytes. Cells were exposed to a dose of UV-B radiation (15.6W/m$^2$) and to evaluate the degree of DNA damage after UV radiation, the amount of thymine dimers formed were then quantified using specific antibody staining and flow cytometry. Results showed that treatment with exopolysaccharide-protein complex preparations, in a dose-dependent manner, significantly protected from UV-B induced-DNA lesions, by reducing the amount of thymine dimmers.

In a further preferred embodiment, said use of the exopolysaccharide-protein complex as previously defined is in the cosmetic field, in particular for the cosmetic treatment of the skin, more particularly, for promoting the synthesis of keratin. As shown in the example section, endogenous keratin-1 synthesis was determined using a specific antibody in an ELISA assay. Results showed that the treatment of human keratinocyte cells with exopolysaccharide-protein complex, significantly increased KRT1 synthesis.

The effect of the EPCx complex produced by a bacterium, preferably wherein said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.,* upon the global oxidative cell state of human keratinocytes has also been evaluated through a validated metabolomics analytic technique, according to the Food and Drug Administration (FDA) guidelines. Cellular metabolites (reduced and oxidized forms) involved in the cell antioxidant protection system, have been quantified through HPLC/MS-MS; allowing the integrative analysis of the antioxidant global capability with improved accuracy than widely used in vitro antioxidant chemical reactions. EPCx complex has a powerful antioxidant activity, scavenging and detoxifying the cell from free radicals through the metabolic pathways of glutathione and 8-hydroxy-2'deoxyguanosine.

Also provided herein is an *in vitro* toxicity test to evaluate the cytotoxic potential of EPCx on cell lines. The cytotoxicity evaluation of each concentration of EPCx was performed by using the MTT Cell Proliferation Assay in order to determine the concentrations which are not harmful to Human Epidermal Keratinocytes (HEK). As shown in the example section, the EPCx show *in vitro*

Also provided herein is a cutaneous compatibility assessment of EPCx under dermatological control for single patch application in human volunteers (Patch test). The result showed that the composition was non-irritating and has a very good cutaneous compatibility.

The present invention further relates to the use of at least one exopolysaccharide-protein complex obtained from a bacterium, wherein said exopolysaccharide-protein complex comprises or consist of: (i) a crude exopolysaccharide, and (ii) exopolysaccharide-associated proteins as an antioxidant agent, preferably wherein said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp..* Preferably, said at least one exopolysaccharide-protein complex for use as antioxidant agent is included in a pharmaceutical, a cosmetic, a cosmeceutical, a dermatologic or food composition.

The terms "comprising" or "comprise(s)"or "consisting of" or "consists of" are as previously defined.

In a preferred embodiment, the crude exopolysaccharide contained in said exopolysaccharide-protein complex obtained from a bacterium for use as antioxidant agent, is a heteropolymer comprising or consisting of:

- glucose units,
- galactose units,
- uronic acid units, and
- other sugar units selected from rhamnose, glucosamine and a mixture of rhamnose and glucosamine.

In a further preferred embodiment, the crude exopolysaccharide contained in said exopolysaccharide-protein complex obtained from a bacterium for use as an antioxidant agent, comprises or consists of: (a) 30 to 60 wt% glucose, (b) 30 to 50 wt% galactose, (c) 5 to 10 wt % uronic acids and (d) 1 to 10 wt% of other sugar units, providing that the sum of the components in the crude exopolysaccharide is 100 wt%, i.e. the components (a), (b), (c) and (d) must amount 100 wt% if components (a), (b), (c) and (d) are the only components in the exopolysaccharide-protein complex or the components (a), (b), (c) and (d) plus any further component(s) must amount 100 wt% if components (a), (b), (c) and (d) are not the only components in the exopolysaccharide-protein complex.

In a further preferred embodiment, the crude exopolysaccharide contained in said exopolysaccharide-protein complex obtained from a bacterium for use as an antioxidant agent, further comprises sulphate at a concentration from 2 to 10 wt%.

In a further preferred embodiment, in the exopolysaccharide-protein complex for use as an antioxidant agent, the exopolysaccharide-associated proteins include two bands of molecular weight as obtained by SDS-PAGE. In particular, the EPCx has a protein profile comprising at least, according to the SDS-PAGE technique, 12 detectable bands, including two principal bands corresponding, respectively, to molecular weights (approximate molecular weights given in relation to molecular standards, notably provided by Bio-Rad Laboratories) ranging between:

- band 1: 30 kDa and 40 kDa, in particular 33kDa;
- band 2: 51 kDa and 60 kDa, in particular 55kDa;

In a further preferred embodiment, said composition comprising or consisting of the exopolysaccharide-protein complex obtained from a bacterium for use as an antioxidant agent, is used for preventing or treating a subject affected by an oxidative stress-related disease selected from a neurodegenerative pathology, an ischaemic pathology or an inflammatory process. Preferably, said oxidative stress-related disease is selected from skin cancer, skin fibrosis, atopic dermatitis and atopic eczema.

In a further preferred embodiment, said composition comprising or consisting of the exopolysaccharide-protein complex obtained from a bacterium for use as an antioxidant agent is an antiaging composition.

It is also disclosed herein a method of preparing an exopolysaccharide-protein complex according to the first aspect of the invention, including all the embodiments comprised in said first aspect.

The method of preparing the exopolysaccharide-protein complex as disclosed above comprises the steps of:

- culturing a bacterium, preferably said bacterium being from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp,* isolated from a hypersaline environment.
- isolating the exopolysaccharide-protein complex from the secreted fraction of the culture.

The medium suitable to cultivate thebacterium, preferably said bacterium being from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp,* includes a synthetic medium comprising a carbon source selected from the group consisting of: lactose, maltose, glucose, galactose, sucrose, glycerol and mixtures thereof. Preferably, the carbon source is selected from the group consisting of: glucose, lactose, sucrose, and mixtures thereof. In a more preferred embodiment, the carbon source is lactose.

Preferably, the other fermentation medium components are: potassium phosphate dibasic ($K_2HPO_4$), 0.5 wt% - 1.0 wt%; potassium phosphate monobasic ($KH_2PO_4$), 0.1wt % - 0.5wt %; sodium chloride (NaCl),5.0 wt % - 10.0 wt %; magnesium sulfate heptahydrate ($MgSO_4 \cdot 7H_2O$), 0.01 wt % - 0.05 wt %; ammonium sulfate ($NH_4)_2SO_4$, 0.05wt % - 0.5 wt % and peptone, 0.02 wt % - 0.1 wt %.

Preferably, before culturing bacteria, the suitable medium for culturing is a synthetic medium with a pH value ranging from 6 to 8. In a more preferred embodiment, the suitable medium has a pH value of 7.

Preferably, the culture step is conducted in fermenters operating at a temperature ranging from 25°C to 42°C. In a more preferred embodiment, the temperature is 32°C.

Preferably, the fermentation process comprises a fermentation step allowing to grow a strain of the corresponding bacteria, preferably said bacteria being from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp,* in a suitable medium in a fermenter under conditions of agitation sufficient to maintain a homogenous culture and limited aeration such that dissolved oxygen pressure (pO2) within the culture is around 20 to 40 % for most of the fermentation step. Preferably, pO2 within the culture is 30 % in the fermentation step.

As mentioned above, the process of preparing an exopolysaccharide-protein complex comprises the step of isolating the exopolysaccharide-protein complex from the secreted fraction of the culture. Said isolation can be carried by removing other molecules present in the culture media by alcohol precipitation. Non-limiting examples of alcohols which can be used include ethanol, isopropanol, and methanol. In particular, the isolation and purification of the EPCx after alcohol precipitation can be conducted by tangential flow filtration methods using ultrafiltration membranes. Preferably, said membranes have a MWCO of 30 kDa and the retentate recovered after the ultrafiltration comprises the exopolysaccharide and the EPS-associate proteins which can be recovered. The tangential flow filtration can act to both diafilter and concentrate the EPCx.

Accordingly, the inventors have surprisingly and unexpectedly found that the non-toxic and biodegradable exopolysaccharide-protein complex secreted by a bacterium, preferably wherein said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.,* in accordance with the invention, can be used as functional ingredient in skin and/or hair care formulations to prevent and modulate oxidative skin damages and as hair restorer ingredient or in antioxidant formulations. EPCx produced by a bacterium, preferably wherein said bacterium is from a genus selected from *Halomonas, Pseudoalteromonas, Vibrio, Salinivibrio, Marinomonas, Alteromonas, Pseudomonas, Halobacillus, and Bacillus,* more preferably from genus *Halomonas,* still more preferably from *Halomonas elongata sp.,* counteracts the malignant effects of UV irradiation and has a powerful antioxidant activity, scavenging and detoxifying the cell from free radicals through the metabolic pathways of glutathione and 8-hydroxy-2'deoxyguanosineand at the same time increases endogenous keratin synthesis.

The present invention will be more clearly understood with the help of the following examples, without being the present invention limited thereto and included only for illustrative purposes only, showing isolation and characterization of bacteria, the preparation and characterization of EPCx and assays for biological activities in accordance with the invention.

**EXAMPLES**

Example 1. Preparation and Isolation of the Exopolysaccharide-Protein Complex (EPCx) excreted by *Halomonas elongata* Cx.

a) *Method of Culturing of bacterium Halomonas elongata*

The strain of the species *Halomonas elongata* was cultured in a fermenter, at 32°C and at a pH of 7.5, whose broth contained (g $L^{-1}$): 100 NaCl; 50 Lactose; 7 $K_2HPO4$; 2 $KH_2PO4$; 0.1 $MgSO4·7H_2O$; 1 $(NH_4)_2SO_4$ and 0.5 Peptone. An inoculum was prepared with 10% (v/v) of a pre-culture and the duration of the fermentation was extended to 72 hours. The reactors are operated in batch mode, and dissolved oxygen was controlled by the agitation (300 to 900 rpm) at $pO_2$ ≈ 30%.

b) *Purification of EPCx*

The bacterium was separated from the broth by centrifugation at 12,000 g for 45 min. The resulting clear solution was subjected to ultrafiltration and dialysis using an installation for ultrafiltration (Sartocon® SliceCassette, Sartorius Stedim), membrane exclusion limit 30 KDa. If necessary, the final solution may be lyophilized and purified obtaining an exopolysaccharide-protein complex with a yield of 60-80%.

Example 2. Physical-Chemical Characterization of EPCx Produced by *Halomonas elongata* Cx

The content of neutral and acid monosaccharides of the exopolysaccharide obtained according to Example 1 was determined by a method described by Honda et al.(Honda et al., 1989) and Yang et al. (Yang, Zhao, Wang, Wang, & Mei, 2005; Yang, Zhao, Zhou, et al., 2005).Briefly, the purified polysaccharide sample (1 mg) was hydrolyzed with 1 ml of 2 M trifluoroacetic acid at 120°C for 2 h, derivatized with 1-phenyl-3-methyl-5-pyrazolone, and subsequently analyzed by high-performance liquid chromatography with detection by absorbance monitoring at 245 nm.

The percent relationship of sugars obtained was 30-60% of glucose, 30-50% galactose, 5-10% of glucuronic acid, 1-10% of rhamnose and 1-10% of glucosamine, being the amounts consistent with a total of 100%.

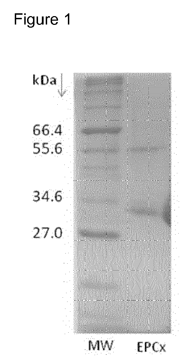

Sodium dodecyl sulfate-polyacrylamide gel electrophoresis (SDS-PAGE) (Laemmli, 1970)was carried out with a 4% stacking and a 9% separating gel. The EPCx sample was dissolved at 10 mg/mL in distilled water and added at 1:3 volume ratio into a buffer solution of 0.5% SDS with 1 %-mercaptoethanol, and then heated to boiling for 5 min. The gels were stained with Coomassie Brilliant Blue R-250 to visualize proteins. 12 detectable bands, including two principal bands of 33 kDa and 55kDa were observed (see figure 1).

Example 3. Effect of exopolysaccharide-protein complex on Human Epidermal Keratinocytes.

The cytotoxicity evaluation of exopolysaccharide-protein complex was performed by using the MTT assay in order to determine the concentrations which are not harmful to Human Epidermal Keratinocytes (HEK). Proliferation of cells lines was measured based on the mitochondria-dependent reduction of yellow tetrazolium MTT (3-(4, 5-dimethylthiazolyl-2)-2, 5- diphenyltetrazolium bromide), to generate reducing equivalents such as NADH and NADPH. Briefly, 5 × $10^3$ cells in 100 μL per well were plated in 96-well tissue culture plates for 24 h. Cells were incubated for 72 h in the presence of different concentrations of EPS (1, 0.1 and 0.01 mg/ml). After that, 20 μL of MTT solution (5 mg/mL in PBS) were added into each well and followed by further incubation for 4-5 h. The resulting intracellular purple formazan can be solubilised and quantified by spectrophotometric means. The results are given in Table 1 below.

Table 1. Effect of exopolysaccharide-protein complex on Human Epidermal Keratinocytes cell line.

| Concentration (mg/ml) | Cell viability % of MTT conversion compared to control |
|---|---|
| 1 | 101 |
| 0.1 | 98 |
| 0.01 | 102 |

No viability alteration and no significant changes in Human Epidermal Keratinocytes proliferation was observed at the exopolysaccharide-protein complex concentrations tested in this assay.

Example 4. <u>Cutaneous compatibility assessment of exopolysaccharide-protein complex under dermatological control (Patch test).</u>

The irritation potential of exopolysaccharide-protein complex was evaluated, after a single application under exaggerated experimental conditions. The product was applied, only once, over the skin of the back and under occlusive patch. The compatibility of EPCx with the skin was verified, after 15 minutes of removing the patches and by means of visual exam of the experimental area.

Ten subjects entered into the study, Subjects were not currently taking any medications including anti-inflammatory agents, antibiotics, and/or anti-histamines. Subjects were not pregnant or nursing and were between the ages of 18-70. The subjects had no known allergies to other skin care products. Subjects were instructed not to apply any other products to the test area. Subjects were instructed not to use body scrubs or exfoliants, washcloths, loofahs, or sponges on the test area. Subjects were instructed not to swim, use a sauna, or receive sun exposure at tanning salons on their back for the duration of the study. Subjects were permitted to shower, but were instructed to avoid exposing the test area to direct streams of water or excessive soaking.

EPCx was applied on the patch on the top of the back in occlusive conditions for 48 hours. One patch without EPCx was applied in the same experimental conditions. Skin reactions were evaluated after patch removal according to the scores reported in table 2, which describes the severity of Erythema (E) and Oedema (OE) parameters.

Table 2. Clinical examination and scoring.

| Score | Assessment of reaction | Parameters evaluated | |
|---|---|---|---|
| | | Erythema (E) | Oedema (OE) |
| -:0 | Absence | No erythema | No oedema |
| ±:0.5 | Doubtful | Very slight erythema (barely perceptible: quiet pinked coloration of one part of the tested area | - |
| +:1 | Slight | slight erythema (quiet pinked coloration of the completed or rather visible on one part of the tested area) | Slight oedema (palpable and visible) |
| ++:2 | Moderate | Obvius erythema (clear erythema covering all of tested area) | Obvius oedema with or without papule/s or vesicle/s |
| +++:3 | High | Intense erythema (severe erythema covering all the tested area or erythema diffusing outside the tested area | Intense oedema (extended area outside the tested area) with or without vesicle/s or blister/s |

The analysis and the interpretation of the result were carried out by calculation of the Mean Irritation Index (MII).

$$MII = \sum \text{of erythema and oedema degree/Number of volunteers}.$$

MII was used to classify EPCx according to the following scale (Table 3):

Table 3. Classification according MII.

| MII | Product classification |
|---|---|
| MII=0.0 | Non Irritating (NI)/ Very Good Cutaneous Compatibility |
| MII<0.20 | Non Irritating (NI)/ Good Cutaneous Compatibility |
| $0.20 \leq MII < 0.50$ | Slightly Irritating (SI)/ Intermediate Cutaneous Compatibility |
| $0.50 \leq MII < 1$ | Moderately Irritating (MI)/ Bad Cutaneous Compatibility |
| $MII \geq 1$ | Irritating (I)/ Very bad Cutaneous Compatibility |

According to the methodology used to assess the potential for causing irritability of EPCx product, it may be concluded that the composite does not cause any cutaneous irritation or sensitization, during the trial period (MII=0.00).

EPCx is non-irritating and has a very good cutaneous compatibility.

Example 5. Protective effects of exopolysaccharide-protein complex on DNA lesions induced by UV

Human keratinocytes cells were cultured overnight at 100.000 cells/well density in a 24 well plate. After 24 hours, the culture media was removed and was substituted for new culture medium with the compound at different concentrations (1 or 10 $\mu$g/ml) for 4 hours. Medium for control replicates were replaced by fresh medium without product. After treatment, medium was replaced by PBS in plates for irradiation and plates were irradiated with UVB (15.6 W/m$^2$) for 5 minutes. Non-irradiated control group was not irradiated. Immediately after irradiation, cells were disaggregated with T/E at 37 °C, fixed with 4% PFA and processed for quantification of thymine dimers.

For thymine dimer measurement. Fixed cells were blocked with 10% FBS in PBS. After blocking step, cells were incubated with Anti-Thymine Dimer Antibody at a 1/250 dilution. overnight at 4 °C. After incubation with primary antibody cells were centrifuged and washed three time with PBS, and incubated with polyclonal secondary antibody to mouse 488 Alexa Ig G2. Finally, stained cells were centrifuged and washed three times with PBS, resuspended in 500 $\mu$L of PBS, and analyzed on flow cytometer.

Data were represented normalized to "Control UV" and the data defined the efficacy of the irradiation inducing Thymine Dimers (comparing Control without UV vs Control UV) and the efficacy of the treatment preventing UVB damage.

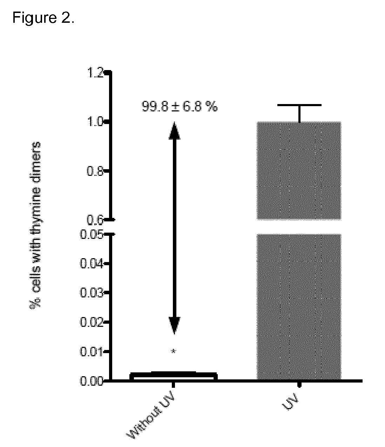

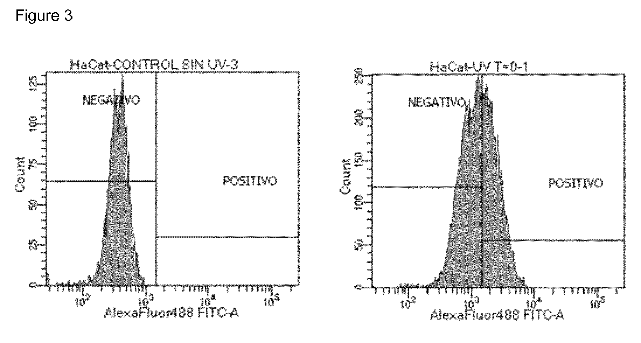

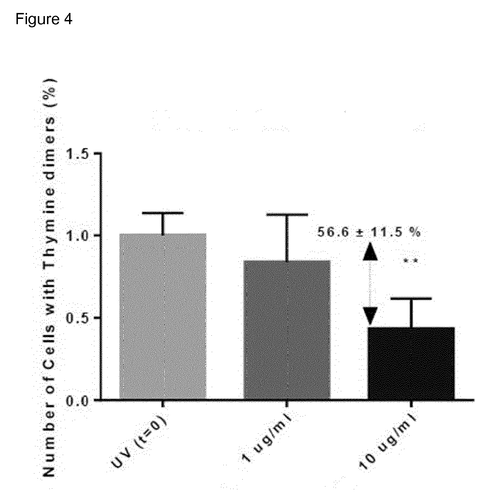

Results showed that the applied protocol of UVB irradiation increased DNA lesions in form of thymine dimers by 99.8 $\pm$ 6.8 %, compared to the non-irradiated control group, immediately after irradiation (figure 2 and figure 3) and the treatment with EPCx before irradiation significantly protected from UV-B induced-thymine dimers formation. The percentage of cells presenting thymine dimers was significantly reduced in cells treated with 10 $\mu$g/ml. The reduction of thymine dimers was 56.6 $\pm$ 11.5 % lower in keratinocytes previously treated with the EPCx than in untreated group (Figure 4).

The effect upon the global oxidative cell state of human keratinocytes has been evaluated through a validated metabolomics analytic technique, according to the Food and Drug Administration (FDA) guidelines. 10 cellular metabolites (reduced and oxidized forms) involved in the cell antioxidant protection system, have been quantified through HPLC/MS-MS; allowing the integrative analysis of the antioxidant global capability with improved accuracy than widely used in vitro antioxidant chemical reactions (TAC, ORAC...).

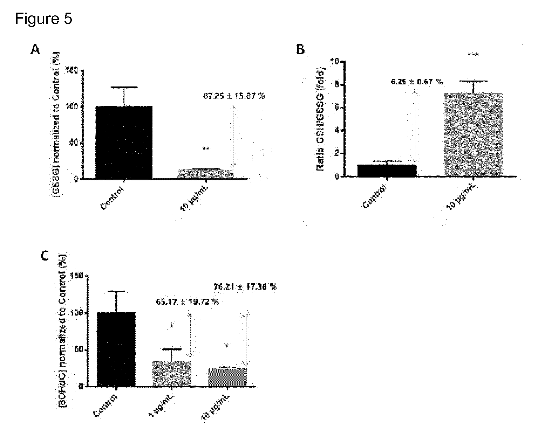

As shown in Figure 5A, EPCx at the concentration of 10 $\mu$g/ml significantly reduced the oxidized form of glutathione, GSSG (Glutathione disulphide), by 87.25 $\pm$ 15.87 %, without decreasing the reduced form, GSH (Glutathione). Thus, ratio GSH/GSSG (Figure 5B), biomarker of oxidative stress, is increased in a 6.25 $\pm$ 0.67 fold. EPCx also showed activity upon the 8OHdG(8-hydroxy-2'deoxyguanosine), a critical biomarker of oxidative stress and carcinogenesis. EPCx significantly reduced this metabolite in 65.17 $\pm$ 19.72 and 76.21 $\pm$ 17.36, when added to keratinocytes at concentrations of 1 $\mu$g/ml and 10 $\mu$g/ml respectively (Figure 5C).

Example 6. Effect of exopolysaccharide-protein complex on KRT1 (keratin 1) synthesis in human keratinocyte cells.

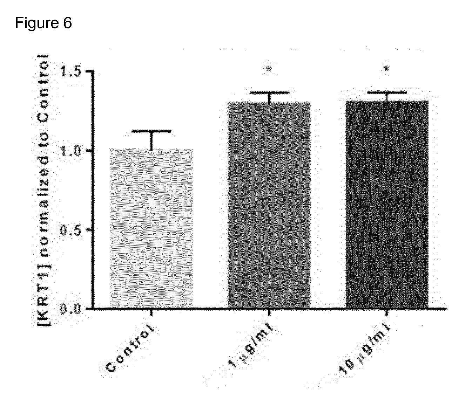

The capacity of EPCx to induce keratin synthesis was evaluated *in vitro* in human keratinocytes. Human keratinocytes were cultured overnight at 200.000 cells/well density in a 6-well plate (6 replicates (wells) per treatment and 6 replicates for the control). After 24 h, exopolysaccharide was added at final concentrations of 1 $\mu$g/ml and 10 $\mu$g/ml, while nothing was added to the control cells. Cells were incubated for 24 h before total protein was extracted for ELISA assay. Treated and control cells were detached with trypsin and then collected by centrifugation, washed 3 times with cold PBS buffer and resuspended in PBS. Cells were frozen in liquid nitrogen and after three freeze-thaw cycles, cell debris were removed by centrifugation, and total protein extract (the supernatant) was stored in a new eppendorf tube. Concentration of each total protein extract was determined using the BCA assay. Sample concentration was normalized in order to load the same amount of total protein in the following assay (ELISA- Enzyme-Lynked-Immuno-Sorbent Assay). Each extract was incubated with the Human antibody against KRT1, coated onto the wells of microtiter strips, by pipetting the volume of protein extract corresponding to 10 □g. The ELISA protocol works with a specific primary antibody binding to the KRT1 in the sample, and then a second antibody biotinylated binding to the HuKRT1-antibody. A third compound (streptavidin-peroxidase) recognizes the biotinylated antibody. Finally, a substrate for the streptavidin-peroxidase is added, to produce a colored precipitate. The intensity of the colored product (450 nm) is directly proportional to the concentration of KRT1 present in the sample. KRT1 expression was evaluated using 1 $\mu$g/ml and 10 $\mu$g/ml concentrations of EPCx in the treated samples. The concentration of KRT1 (Treated vs Control) was represented graphically by calculating the mean value among the control biological replicates and the mean value of the treated biological replicates (figure 6). The results show that treatment with EPCx at 1 $\mu$g/ml and 10 $\mu$g/ml concentrations, increased KRT1 synthesis by 29.0 $\pm$ 8.5 % and 29.8 $\pm$ 8.3 % respectively, when compared with untreated control cells.

Human keratinocyte cells were incubated with EPCx at 1 $\mu$g/ml and 10 $\mu$g/ml concentrations. After an incubation period of 24 h, total proteins were purified and quantified. KRT1 synthesis was determined using a specific antibody in an ELISA assay in treated and control (untreated) cells.

**REFERENCES**

Cambon-Bonavita, M. A., Raguenes, G., Jean, J., Vincent, P., & Guezennec, J. (2002). A novel polymer produced by a bacterium isolated from a deep-sea hydrothermal vent polychaete annelid. Journal ofApplied Microbiology, 93(2), 310-315.

CHENG, K., CAHILL, D., KASAI, H., NISHIMURA, S., & LOEB, L. (1992). 8-HYDROXYGUANINE, AN ABUNDANT FORM OF OXIDATIVE DNA DAMAGE, CAUSES G -> T AND A -> C SUBSTITUTIONS. [Article]. Journal of Biological Chemistry, 267(1), 166-172.

Costa, R. R., Neto, A. I., Calgeris, I., Correia, C. R., Pinho, A. C. M., Fonseca, J., et al. (2013). Adhesive nanostructured multilayer films using a bacterial exopolysaccharide for biomedical applications. Journal of Materials Chemistry B, 1(18), 2367-2374.

Courtois, A., Thollas, B., Delgado, R., Cebrian, J., & Soley, A. (2012). WO Patent No. WO 2012/072245 A2.

Donot, F., Fontana, A., Baccou, J. C., & Schorr-Galindo, S. (2012). Microbial exopolysaccharides: Main examples of synthesis, excretion, genetics and extraction. Carbohydrate Polymers, 87(2), 951-962.

GarcÍA Sanz, M. N., Ferrer Montiel Antonio, V., Soley Astals, A., & AlmiÑAna DomÉNech, N. (2015). WO Patent No. WO 2015/063240 A1.

Genestra, M. (2007). Oxyl radicals, redox-sensitive signalling cascades and antioxidants. [Review]. Cellular Signalling, 19(9), 1807-1819.

Gorska, S., Schwarzer, M., Jachymek, W., Srutkova, D., Brzozowska, E., Kozakova, H., et al. (2014). Distinct Immunomodulation of Bone Marrow-Derived Dendritic Cell Responses to Lactobacillus plantarum WCFS1 by Two Different Polysaccharides Isolated from Lactobacillus rhamnosus LOCK 0900. Applied and Environmental Microbiology, 80(20), 6506-6516.

Honda, S., Akao, E., Suzuki, S., Okuda, M., Kakehi, K., & Nakamura, J. (1989). HIGH-PERFORMANCE LIQUID-CHROMATOGRAPHY OF REDUCING CARBOHYDRATES AS STRONGLY ULTRAVIOLET-ABSORBING AND ELECTROCHEMICALLY SENSITIVE 1-PHENYL-3-METHYL-5-PYRAZOLONE DERIVATIVES. Analytical Biochemistry, 180(2), 351-357.

Laemmli, U. K. (1970). CLEAVAGE OF STRUCTURAL PROTEINS DURING ASSEMBLY OF HEAD OF BACTERIOPHAGE-T4. Nature, 227(5259), 680-&.

Loing, E., Briatte, S., Vayssier, C., Beaulieu, M., & Dionne, P. (2009). WO Patent No. WO 2009/127057 A1.

Maini, S., Fahlman, B. M., & Krol, E. S. (2015). Flavonols Protect Against UV Radiation-Induced Thymine Dimer Formation in an Artificial Skin Mimic. Journal of Pharmacy and Pharmaceutical Sciences, 18(4), 600-615.

MercadÉ Gil Mª, E., CarriÓN Fonseca, O., & Montes LOPez Mª, J. (2015). WO Patent No. WO 2015/117985 A1.

Mishra, A. K., Mishra, A., & Chattopadhyay, P. (2011). Herbal Cosmeceuticals for Photoprotection from Ultraviolet B Radiation: A Review. Tropical Journal of Pharmaceutical Research, 10(3), 351-360.

Nevot, M., Deroncele, V., Lopez-Iglesias, C., Bozal, N., Guinea, J., & Mercade, E. (2006). Ultrastructural analysis of the extracellular matter secreted by the psychrotolerant bacterium Pseudoalteromonas antarctica NF3. Microbial Ecology, 51(4), 501-507.

Nevot, M., Deroncele, V., Messner, P., Guinea, J., & Mercade, E. (2006). Characterization of outer membrane vesicles released by the psychrotolerant bacterium Pseudoalteromonas antarctica NF3. Environmental Microbiology, 8(9), 1523-1533.

Oresajo, C., Krutmann, J., Yatskayer, M., & Grether-Beck, S. (2012). Effect of an antioxidant combination on infrared-a induced MMP1-upregulation in human dermal fibroblasts. Journal of the American Academy of Dermatology, 66(4), AB 175-AB 175.

Pauloin, T., Dutot, M., Joly, F., Warnet, J., & Rat, P. (2009). High molecular weight hyaluronan decreases UVB-induced apoptosis and inflammation in human epithelial corneal cells. [Article]. Molecular Vision, 15(57-58), 577-583.

Poli, A., Anzelmo, G., & Nicolaus, B. (2010). Bacterial Exopolysaccharides from Extreme Marine Habitats: Production, Characterization and Biological Activities. Marine Drugs, 8(6), 1779-1802.

Pourzand, C., & Tyrrell, R. (1999). Apoptosis, the role of oxidative stress and the example of solar UV radiation. [Review]. Photochemistry and Photobiology, 70(4), 380-390.

Radchenkova, N., Panchev, I., Vassilev, S., Kuncheva, M., Dobreva, S., & Kambourova, M. (2015). Continuous cultivation of a thermophilic bacterium Aeribacillus pallidus 418 for production of an exopolysaccharide applicable in cosmetic creams. Journal of Applied Microbiology, 119(5), 1301-1309.

Shao, L., Wu, Z., Zhang, H., Chen, W., Ai, L., & Guo, B. (2014). Partial characterization and immunostimulatory activity of exopolysaccharides from Lactobacillus rhamnosus KF5. Carbohydrate Polymers, 107, 51-56.

Woelfle, U., Esser, P. R., Simon-Haarhaus, B., Martin, S. F., Lademann, J., & Schempp, C. M. (2011). UVB-induced DNA damage, generation of reactive oxygen species, and inflammation are effectively attenuated by the flavonoid luteolin in vitro and in vivo. Free Radical Biology and Medicine, 50(9), 1081-1093.

Yang, X. B., Zhao, Y., Wang, Q. W., Wang, H. F., & Mei, Q. B. (2005). Analysis of the monosaccharide components in Angelica polysaccharides by high performance liquid chromatography. Analytical Sciences, 21(10), 1177-1180.

Yang, X. B., Zhao, Y., Zhou, S. Y., Liu, L., Wang, H. F., & Mei, Q. B. (2005). Analysis of monosaccharide composition in Angelica polysaccharides by precolumn derivatization high performance liquid chromatography. Chinese Journal of Analytical Chemistry, 33(9), 1287-1290.

**Claims**

1. Use of at least one exopolysaccharide-protein complex obtained from a bacterium, wherein said exopolysaccharide-protein complex comprises: (i) a crude exopolysaccharide, and (ii) exopolysaccharide-associated proteins for the cosmetic treatment of the skin and/or hair.

2. Use of at least one exopolysaccharide-protein complex obtained from a bacterium, wherein said exopolysaccharide-protein complex comprises: (i) a crude exopolysaccharide, and (ii) exopolysaccharide-associated proteins as an antioxidant agent.

3. Use according to claim 1 or 2, wherein the crude exopolysaccharide is an heteropolymer comprising glucose, galactose, uronic acid units and other sugar units selected from rhamnose, glucosamine and a mixture of rhamnose and glucosamine.

4. Use according to any of claims 1 to 3, wherein the crude exopolysaccharide comprises:

   30 to 60 wt% glucose, 30 to 50 wt% galactose, 5 to 10 wt % uronic acids and 1 to 10 wt% of other sugar units, providing that the sum of the components in the crude exopolysaccharide is 100 wt%.

5. Use according to any of claims 1 to 4, wherein the crude exopolysaccharide further comprises sulphate at a concentration from 2 to 10 wt%.

6. Use according to any of claims 1 to 5, wherein the exopolysaccharide-associated proteins include two bands of molecular weight corresponding to a band between 30 and 40 kDa and a band between 51 kDa and 60 kDa as obtained by SDS-PAGE.

7. Use according to any of claims 1 to 6, wherein said bacterium is from the genus selected from *Halomonas, Pseudoalteromonas, Vibrio, Salinivibrio, Marinomonas, Alteromonas, Pseudomonas, Halobacillus, and Bacillus.*

8. Use according to claim 7, wherein said bacterium is from genus *Halomonas.*

9. Use according to claim 8, wherein said bacterium is from *Halomonas elongata sp.*

10. Use according to any of claims 1 and 3 to 9, wherein said cosmetic treatment of the skin is a treatment for protecting the skin from ultraviolet radiation due to sun exposure.

11. Use according to any of claims 1 and 3 to 9, wherein said cosmetic treatment of the hair is for promoting the synthesis of keratin.

12. Use according to any of claims 2 to 9, wherein said at least one exopolysaccharide-protein complex is included in a composition which is a pharmaceutical, a cosmetic, a cosmeceutical, a dermatologic or food composition.

13. Use according to claim 12, wherein said composition is for preventing or treating a subject affected by an oxidative stress-related disease selected from a neurodegenerative pathology, an ischaemic pathology or an inflammatory process.

14. Use according to claim 12 or 13, wherein said composition is for preventing or treating a subject affected by a disease selected from skin cancer, psoriasis, skin fibrosis, atopic dermatitis and atopic eczema.

**15.** Use according to any of claims 2 to 9, wherein said at least one exopolysaccharide-protein complex is included in an antiaging composition.

Figure 1

Figure 2.

Figure 3

Figure 4

Figure 5

Figure 6

Europäisches
Patentamt

European
Patent Office

Office européen
des brevets

# EUROPEAN SEARCH REPORT

Application Number

EP 16 38 2437

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/067218 A1 (LUBRIZOL ADVANCED MAT INC [US]) 6 May 2016 (2016-05-06) | 1,7,8, 10-15 | INV. A61K8/73 |
| Y | * paragraph [0009]; claims 1-15 * | 2-6,9 | A61K35/74 A61Q19/08 |
| X | US 2015/079137 A1 (DELGADO-GONZÁLEZ RAQUEL [ES] ET AL) 19 March 2015 (2015-03-19) * claims 49-69 * | 1-7,9-15 | A61K8/99 |
| X,D | WO 2015/063240 A1 (LIPOTEC S A U [ES]) 7 May 2015 (2015-05-07) * claims 1-12 * | 1,7,8, 10-15 | |
| Y | ANTHONY COURTOIS ET AL: "Exopolysaccharides Isolated from Hydrothermal Vent Bacteria Can Modulate the Complement System", PLOS ONE, vol. 9, no. 4, 15 April 2014 (2014-04-15), page e94965, XP055253215, DOI: 10.1371/journal.pone.0094965 * "Material and Methods", point 2.1; page e94965, left-hand column * | 1-15 | |
| X | CHIN-FENG LIU ET AL: "Immunomodulatory and antioxidant potential of Lactobacillus exopolysaccharides", JOURNAL OF THE SCIENCE OF FOOD AND AGRICULTURE, 1 January 2011 (2011-01-01), pages n/a-n/a, XP055316627, GB ISSN: 0022-5142, DOI: 10.1002/jsfa.4456 | 2 | |
| Y | * abstract * * page 2285, left-hand column, paragraph 1-2 * * figure 7 * | 1,3-15 | |

TECHNICAL FIELDS SEARCHED (IPC)

A61K
A61Q

The present search report has been drawn up for all claims

| Place of search | Date of completion of the search | Examiner |
|---|---|---|
| Munich | 16 November 2016 | Hörtner, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 295 927 A1

## ANNEX TO THE EUROPEAN SEARCH REPORT
## ON EUROPEAN PATENT APPLICATION NO.

EP 16 38 2437

16-11-2016

| Patent document cited in search report | Publication date | Patent family member(s) | | Publication date |
|---|---|---|---|---|
| WO 2016067218 A1 | 06-05-2016 | NONE | | |
| US 2015079137 A1 | 19-03-2015 | AU | 2013237387 A1 | 02-10-2014 |
| | | EP | 2827837 A2 | 28-01-2015 |
| | | ES | 2424294 A1 | 30-09-2013 |
| | | JP | 2015510919 A | 13-04-2015 |
| | | KR | 20150005928 A | 15-01-2015 |
| | | US | 2015079137 A1 | 19-03-2015 |
| | | WO | 2013139965 A2 | 26-09-2013 |
| WO 2015063240 A1 | 07-05-2015 | CN | 105934231 A | 07-09-2016 |
| | | EP | 2868308 A1 | 06-05-2015 |
| | | EP | 3062759 A1 | 07-09-2016 |
| | | KR | 20160078458 A | 04-07-2016 |
| | | US | 2016263014 A1 | 15-09-2016 |
| | | WO | 2015063240 A1 | 07-05-2015 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012072245 A2
- WO 2016066857 A1
- WO 2015117985 A1
- WO 2015063240 A1
- WO 2009127057 A1

**Non-patent literature cited in the description**

- **CAMBON-BONAVITA, M. A. ; RAGUENES, G. ; JEAN, J. ; VINCENT, P. ; GUEZENNEC, J.** A novel polymer produced by a bacterium isolated from a deep-sea hydrothermal vent polychaete annelid. *Journal ofApplied Microbiology,* 2002, vol. 93 (2), 310-315
- **CHENG, K. ; CAHILL, D. ; KASAI, H. ; NISHIMURA, S. ; LOEB, L.** 8-HYDROXYGUANINE, AN ABUNDANT FORM OF OXIDATIVE DNA DAMAGE, CAUSES G -> T AND A -> C SUBSTITUTIONS. *Journal of Biological Chemistry,* 1992, vol. 267 (1), 166-172
- **COSTA, R. R. ; NETO, A. I. ; CALGERIS, I. ; CORREIA, C. R. ; PINHO, A. C. M. ; FONSECA, J. et al.** Adhesive nanostructured multilayer films using a bacterial exopolysaccharide for biomedical applications. *Journal of Materials Chemistry B,* 2013, vol. 1 (18), 2367-2374
- **DONOT, F. ; FONTANA, A. ; BACCOU, J. C. ; SCHORR-GALINDO, S.** Microbial exopolysaccharides: Main examples of synthesis, excretion, genetics and extraction. *Carbohydrate Polymers,* 2012, vol. 87 (2), 951-962
- **GENESTRA, M.** Oxyl radicals, redox-sensitive signalling cascades and antioxidants. *Cellular Signalling,* 2007, vol. 19 (9), 1807-1819
- **GORSKA, S. ; SCHWARZER, M. ; JACHYMEK, W. ; SRUTKOVA, D. ; BRZOZOWSKA, E. ; KOZAKOVA, H. et al.** Distinct Immunomodulation of Bone Marrow-Derived Dendritic Cell Responses to Lactobacillus plantarum WCFS1 by Two Different Polysaccharides Isolated from Lactobacillus rhamnosus LOCK 0900. *Applied and Environmental Microbiology,* 2014, vol. 80 (20), 6506-6516
- **HONDA, S. ; AKAO, E. ; SUZUKI, S. ; OKUDA, M. ; KAKEHI, K. ; NAKAMURA, J.** HIGH-PERFORMANCE LIQUID-CHROMATOGRAPHY OF REDUCING CARBOHYDRATES AS STRONGLY ULTRAVIOLET-ABSORBING AND ELECTROCHEMICALLY SENSITIVE 1-PHENYL-3-METHYL-5-PYRAZOLONE DERIVATIVES. *Analytical Biochemistry,* 1989, vol. 180 (2), 351-357
- **LAEMMLI, U. K.** CLEAVAGE OF STRUCTURAL PROTEINS DURING ASSEMBLY OF HEAD OF BACTERIOPHAGE-T4. *Nature,* 1970, vol. 227 (5259), 680
- **MAINI, S. ; FAHLMAN, B. M. ; KROL, E. S.** Flavonols Protect Against UV Radiation-Induced Thymine Dimer Formation in an Artificial Skin Mimic. *Journal of Pharmacy and Pharmaceutical Sciences,* 2015, vol. 18 (4), 600-615
- **MISHRA, A. K. ; MISHRA, A. ; CHATTOPADHYAY, P.** Herbal Cosmeceuticals for Photoprotection from Ultraviolet B Radiation: A Review. *Tropical Journal of Pharmaceutical Research,* 2011, vol. 10 (3), 351-360
- **NEVOT, M. ; DERONCELE, V. ; LOPEZ-IGLESIAS, C. ; BOZAL, N. ; GUINEA, J. ; MERCADE, E.** Ultrastructural analysis of the extracellular matter secreted by the psychrotolerant bacterium Pseudoalteromonas antarctica NF3. *Microbial Ecology,* 2006, vol. 51 (4), 501-507
- **NEVOT, M. ; DERONCELE, V. ; MESSNER, P. ; GUINEA, J. ; MERCADE, E.** Characterization of outer membrane vesicles released by the psychrotolerant bacterium Pseudoalteromonas antarctica NF3. *Environmental Microbiology,* 2006, vol. 8 (9), 1523-1533
- **ORESAJO, C. ; KRUTMANN, J. ; YATSKAYER, M. ; GRETHER-BECK, S.** Effect of an antioxidant combination on infrared-a induced MMP1-upregulation in human dermal fibroblasts. *Journal of the American Academy of Dermatology,* 2012, vol. 66 (4), AB 175-AB 175
- **PAULOIN, T. ; DUTOT, M. ; JOLY, F. ; WARNET, J. ; RAT, P.** High molecular weight hyaluronan decreases UVB-induced apoptosis and inflammation in human epithelial corneal cells. *Molecular Vision,* 2009, vol. 15 (57-58), 577-583
- **POLI, A. ; ANZELMO, G. ; NICOLAUS, B.** Bacterial Exopolysaccharides from Extreme Marine Habitats: Production, Characterization and Biological Activities. *Marine Drugs,* 2010, vol. 8 (6), 1779-1802

- **POURZAND, C. ; TYRRELL, R.** Apoptosis, the role of oxidative stress and the example of solar UV radiation. *Photochemistry and Photobiology,* 1999, vol. 70 (4), 380-390
- **RADCHENKOVA, N. ; PANCHEV, I. ; VASSILEV, S. ; KUNCHEVA, M. ; DOBREVA, S. ; KAMBOUROVA, M.** Continuous cultivation of a thermophilic bacterium Aeribacillus pallidus 418 for production of an exopolysaccharide applicable in cosmetic creams. *Journal of Applied Microbiology,* 2015, vol. 119 (5), 1301-1309
- **SHAO, L. ; WU, Z. ; ZHANG, H. ; CHEN, W. ; AI, L. ; GUO, B.** Partial characterization and immunostimulatory activity of exopolysaccharides from Lactobacillus rhamnosus KF5. *Carbohydrate Polymers,* 2014, vol. 107, 51-56
- **WOELFLE, U. ; ESSER, P. R. ; SIMON-HAARHAUS, B. ; MARTIN, S. F. ; LADEMANN, J. ; SCHEMPP, C. M.** UVB-induced DNA damage, generation of reactive oxygen species, and inflammation are effectively attenuated by the flavonoid luteolin in vitro and in vivo. *Free Radical Biology and Medicine,* 2011, vol. 50 (9), 1081-1093
- **YANG, X. B. ; ZHAO, Y. ; WANG, Q. W. ; WANG, H. F. ; MEI, Q. B.** Analysis of the monosaccharide components in Angelica polysaccharides by high performance liquid chromatography. *Analytical Sciences,* 2005, vol. 21 (10), 1177-1180
- **YANG, X. B. ; ZHAO, Y. ; ZHOU, S. Y. ; LIU, L. ; WANG, H. F. ; MEI, Q. B.** Analysis of monosaccharide composition in Angelica polysaccharides by precolumn derivatization high performance liquid chromatography. *Chinese Journal of Analytical Chemistry,* 2005, vol. 33 (9), 1287-1290